# EUROPEAN PATENT SPECIFICATION

(11) **EP 0 712 468 B1**
(45) Date of publication and mention of the grant of the patent: **15.01.2003**
(21) Application number: 94925198.7
(22) Date of filing: 05.08.1994
(51) Int. Cl.: A61F 5/01, F16F 1/366, F16F 3/12

(54) **COMPOSITE FLEXURE UNIT**
MISCHBIEGUNGSEINHEIT
UNITE DE FLEXION COMPOSITE

(30) Priority: 06.08.1993 US 103197
(43) Date of publication of application: 22.05.1996
(73) Proprietor: Carlson, J. Martin, Edina, MN 55424 (US)
(72) Inventor: Carlson, J. Martin, Edina, MN 55424 (US)
(74) Representative: McLeish, Nicholas Alistair Maxwell
(86) International application number: US9408888
(87) International publication number: WO95004888

(56) References cited:
- CH-A- 320 050
- CH-A- 434 883
- DE-A- 3 834 806
- GB-A- 2 160 779
- GB-A- 2 169 512
- US-A- 1 671 764
- US-A- 1 683 334
- US-A- 1 939 968
- US-A- 4 451 097
- US-A- 4 634 445
- US-A- 4 669 457
- US-A- 5 171 274
- US-A- 5 274 074

## Description

### BACKGROUND OF THE INVENTION

The present invention relates to flexure units, and more particularly, to a composite flexure unit for use as a hinge joint.

Flexure units used for orthoses to provide joint motion, such as for an ankle joint, are typically injection molded polyurethane units. When used in pairs, the flexure units form a joint system with a single axis of rotation passing through both flexure midsections. By choosing good combinations of material properties, size, effective length-to-thickness proportion, and the configuration of the structure to which the flexure units are fastened, the flexure pairs provide relatively free motion about their axis of rotation with a significant blockage of shear, translational and other rotational or undesirable motions.

However, there are some situations where a patient's size, weight impairment and/or ambulation places a loading across the flexure joint which tends to elongate both flexures or elongate one flexure and shorten or compress the other flexure. When used in an orthosis, the elongation can compromise efforts to block joint motion at a given angle, or to allow an unwanted varus or valgus change in the alignment of the orthosis and corresponding anatomy. Also, over time, and with a tension bias through the length of the flexure units, a progressive elongation occurs through stretching and biased movement and/or tearing of the flexure material around its fixation hardware. Thus, it is desirable to increase the longitudinal strength and stiffness of the flexures without increasing flexion stiffness.

Swiss Patent No. 434,883 describes a linkage assembly comprising a flexible connector joining two cylindrical poles. At least one of the poles is provided with a rigid cover rotatably mounted to it.

DE-A-38,34,806 describes a vibration dampening element formed as a flexible strap comprising an intermediate element stretched between two solid elements. The intermediate element is formed from a material that does not break when pulled, such as steel wire or glass fibre and is encased within a material having a low modulus of elasticity, such as lead.

### SUMMARY OF THE INVENTION

The present invention relates to a composite flexure unit comprising a flexure member including a first portion having first mounting means therein for mounting a first end of the flexure member, a second portion having second mounting means therein for mounting a second end of the flexure member, and a third portion connecting the first portion to the second portion, the flexure member being operable for bending about a rotational axis passing through the third portion of the flexure member; and a load bearing element connected between the first and second mounting means; the flexure member having a low modulus of elasticity and the load bearing element having a high modulus of elasticity for providing longitudinal strength and stiffness to the flexure member without significantly increasing flexion stiffness about the rotational axis, the load bearing element bending with the flexure member as the flexure member bends; characterised in that the load bearing element comprises a multi-strand element formed of a fiber having a low coefficient of friction, the fiber being wrapped around and extending between end sleeves forming the mounting means to form two multi-strand lengths of fiber strands in which the fiber strands are free to slide relative to other fiber strands in the same multi-strand length. Bending of the load bearing element with the flexure member may be achieved, for example, by locating the load bearing element in close proximity to or on the neutral plane of bending of the flexure member.

In a preferred embodiment, the load bearing element is positioned in the first and second portions of the flexure member and is a tension carrying member. The tension carrying member can take many forms and a multi-strand element of oriented Teflon fibers has been found satisfactory.

### BRIEF DESCRIPTION OF THE DRAWINGS

Figure 1 is a perspective view of a composite flexure unit showing a first tensile load bearing element;
Figure 2 is a perspective view of a composite flexure unit showing a second tensile load bearing element;
Figure 3 is a perspective view of a composite flexure unit showing a third tensile load bearing element;
Figure 4 is a perspective view of a composite flexure unit showing a fourth tensile load bearing element;
Figure 5 is a perspective view of a composite flexure unit showing a fifth load bearing element;
Figure 6 is a side elevational view of a composite flexure unit with a first cover plate arrangement;
Figure 7 is a side elevational view of a composite flexure unit with a second cover plate arrangement;
Figure 8 is a sectional view taken along line 8--8 of Figure 7;
Figure 9 is a sectional view taken along line 9--9 of Figure 7;
Figure 10 is a front elevational view of the embodiment of Figure 6 showing a first cover plate arrangement;
Figure 11 is a front elevational view of the composite flexure unit showing a second cover plate arrangement; and
Figure 12 is a side elevational view of an embodiment of the present invention.
Figure 13 is a side elevational view of a further embodiment;
Figures 14 through 19 are schematic illustrations of a preferred embodiment of the present invention showing steps of forming a tensile load bearing element from a strand of oriented tetrafluoroethylene;
Figure 20 is a schematic view of a finished tensile load bearing element made as shown in Figures 13 through 19;
Figure 21 is a side view of the element of Figure 20 after encapsulation in a material of low modulus of elasticity;
Figure 22 is a fragmentary sectional view of an orthotic shell having a composite; and
Figure 23 is a sectional view taken on line 23--23 in Figure 22.

### DETAILED DESCRIPTION OF THE PREFERRED EMBODIMENTS

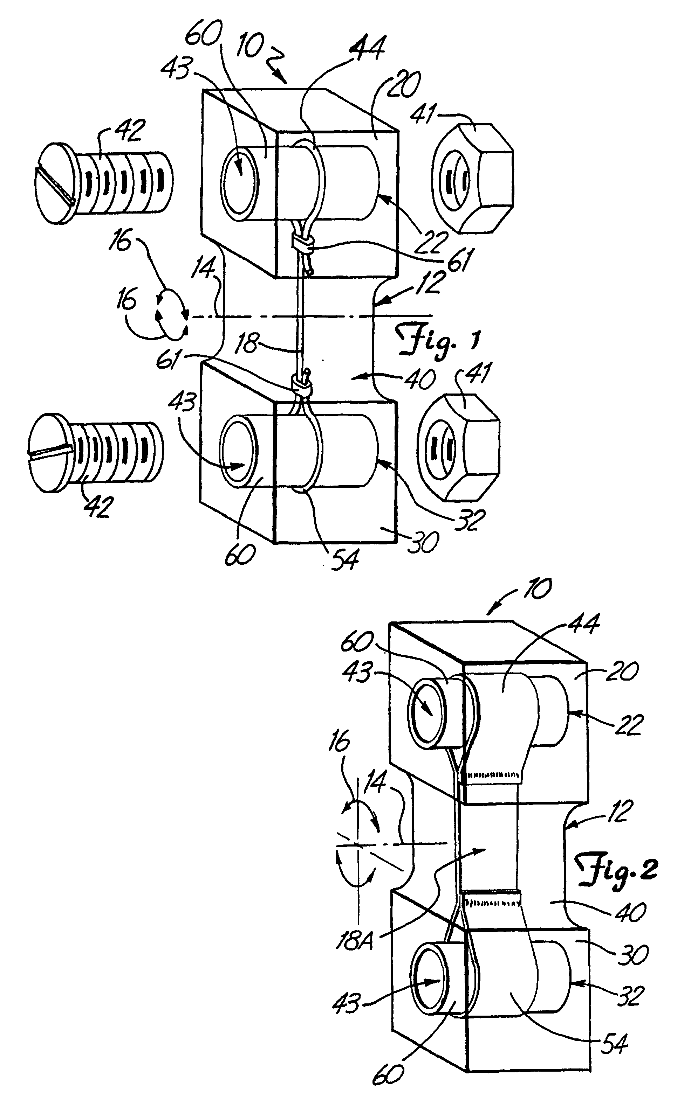

The embodiments of the load bearing element in figures 1 to 5 do not fall under the scope of the appended claims.

Referring to Figure 1, a composite flexure unit, indicated generally at 10, includes a flexure 12 comprised of a low modulus of elasticity material which is operable about a rotational axis 14 which is typical of any axis in the transverse plane and an elongated load bearing element 18 for providing longitudinal strength and stiffness to the flexure 12 without significantly increasing flexion stiffness of the flexure 12 across a longitudinal bending plane, indicated by arrow 16. The bending plane 16 lies perpendicular to axis 14 and parallel to the longitudinal axis of the element 18.

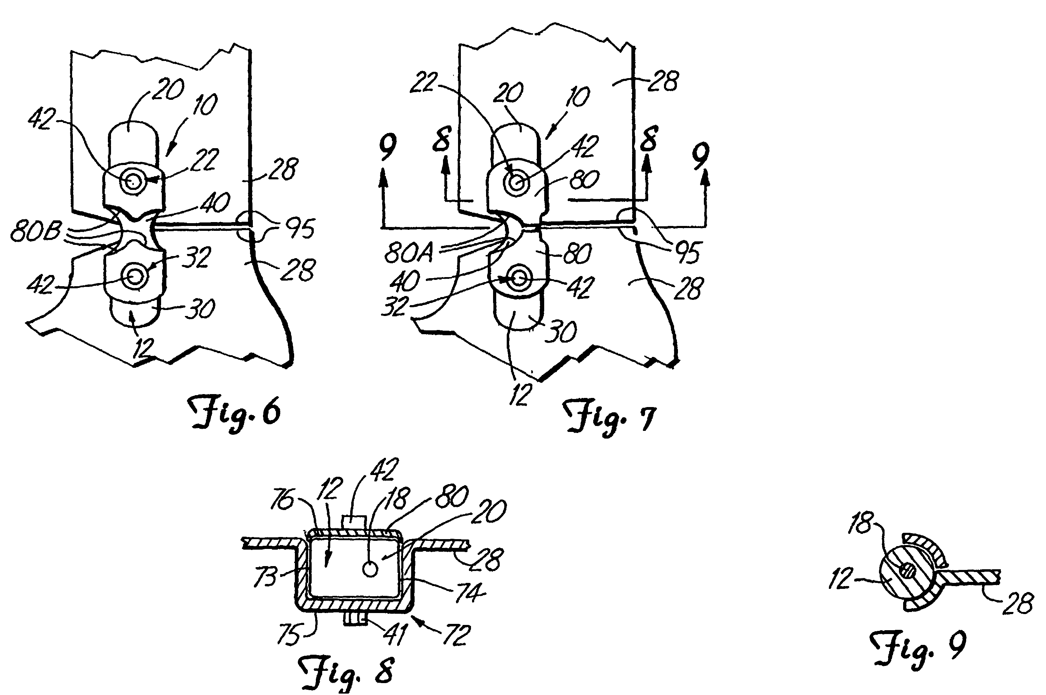

The flexure 12 comprises a column of material having a low modulus of elasticity, such as polyurethane which preferably completely encapsulates the load bearing element 18. However, it is to be understood that encapsulation within the main column of material is not necessary when the load bearing element 18 is a tensile load bearing element. Then, the tension carrying element may be installed to the exterior of the main column material flexure 12 passing through or very close to axis 14 (or to one side of the axis 14 if a bending bias is desired). The flexure 12 includes a first portion 20 having a first mounting device 22 therein for mounting the first portion 20 of the flexure 12 to an orthotic shell 28 (See Figures 6 and 7). A second portion 30 of the flexure 12 includes a second mounting device 32 therein for mounting the second portion 30 of the flexure 12 to the orthotic shell 28. A third portion 40 of the flexure 12 connects the first portion 20 to the second portion 30. The rotational axis 14 is defined through the center of the third portion 40 of the main column of molded material and perpendicular to the longitudinal axis of third portion 40. The longitudinal bending plane 16 is a plane perpendicular to the rotational axis 14 and bisecting the third portion 40 of the flexure 12.

The load bearing element 18 is made of a material having a high modulus of elasticity, such as a metal, glass, or carbon fiber for providing longitudinal strength and stiffness to the flexure unit 10. The load bearing element 18 has a low bending cross-sectional modulus of elasticity, referring to bending it in any plane of its longitudinal axis. In the case of an ankle-foot orthosis, that could be in a direction of dorsiflexion/planterflexion of a patient's ankle. Thus, the load bearing element 18 provides minimal resistance to flexure bending about axis 14. The load bearing element 18 is located in or parallel to the bending plane 16 of the encapsulating flexure 12 and confined to that location to provide a minimal effect on resistance to planterflexions and dorsiflexions. Embedding the slender load bearing element 18 (tension load carrying as shown) within the thicker, stubbier, encapsulating column of material forming flexure 12 in the location noted, preserves the bending compliance of the flexure unit 10 and provides protection to the load bearing element 18. The tensile load bearing element 18 is protected from concentration of shear and bending stresses and from direct blows which might otherwise nick, scratch, or in other ways damage or dislodge the slender load bearing element 18 from its advantageous location.

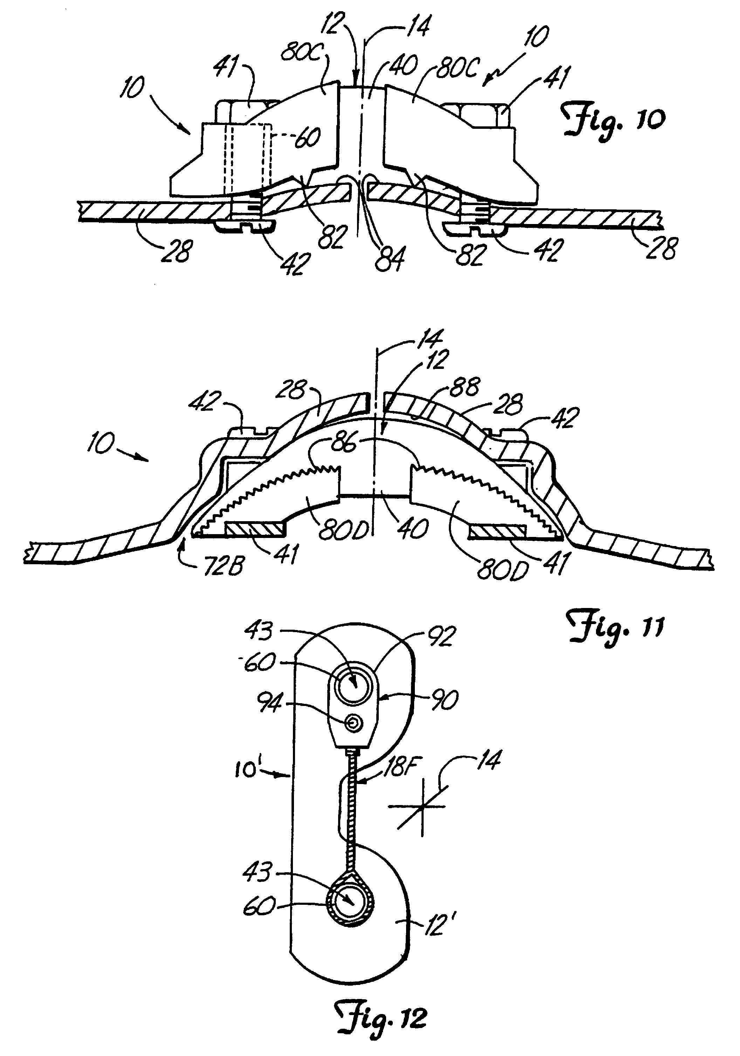

The first and second mounting devices 22 and 32 each include a nut 41, a bolt 42 and possibly, a sleeve 60 having hole 43 through which the bolt 42 passes for fastening the composite flexure 10 to an orthosis shell 28 or other structure (See Figures 10 and 11). It should be apparent to those skilled in the art that other mounting devices may be used, such as, a rod, pin, nail or screw. A first end 44 of the tension load-bearing element 18 is looped around the sleeve 60 of first mounting device 22 and a second end 54 of the load bearing element 18 is looped around the sleeve 60 of the second mounting device 32 such that the load bearing element 18 is secured within the flexure 12.

Each sleeve 60 includes an outer rigid collar 60A and a softer inner collar 60B. The inner collar 60B provides shock absorption while the outer rigid collar 60A spreads the load out across the softer inner collar 60B and insulates the inner collar 60B. The sleeves 60 of the first and second mounting devices 22 and 32 are preferably, but not necessarily, parallel to the rotational axis 14 and perpendicular to the bending plane 16.

When used as orthotic components or in other applications, the composite flexure 10 is sometimes subjected to large repeated tension loads which have the potential of causing fatigue cracking of the tensile load bearing element 18, typically in the area of fixation to the mounting devices 22 and 32. The potential for such fatigue damage and failure depends directly on the magnitude of the peak loading experienced by the load bearing element 18. Also, where the tensile load bearing element 18 mates with the mounting devices 22 and 32, there is a tendency for some rotational and translational relative motion between the tensile load bearing element 18 and the supports such as sleeves 60 which as shown are held by mounting bolts 42. The relative motion causes wear and/or fretting which degrades the mounting and accelerates the fatigue damage. The shock absorbing sleeves 60 positioned between the respective ends of tension bearing element 18 and the bolts 42 or other mounting devices reduces both peak tension loading and the probability of wear. Additional shock absorption may be provided by using a tensile load bearing element 18 which is of extra length and strung loosely between the first and second mounting devices 22 and 32. As the flexure unit 10 is loaded longitudinally the tensile load bearing element 18 is pulled taught to provide shock absorption.

Referring to Figure 1, the load bearing element 18 is shown in the form of a tension cable but may be in the form of a cylindrical filament bundle, wire or rod. A first end 44 of the tensile load bearing element 18 is looped around the shock absorbent sleeve 60 of the first mounting device 22 and fastened to itself by a lock member 61 and a second end 54 of the tensile load bearing element 18 is looped around the shock absorbing sleeve 60 of the second mounting device 32 and fastened to itself by a lock member 61.

Referring to Figure 2, the load bearing element is shown in the form of a very thin tension strap 18A having a wide cross-section with the width dimension being parallel to axis 14 and perpendicular to the bending plane 16 of the orthotic joint. The loop portions 44 and 54 of the strap 18A are sewn (or welded, in the case of a metal strap) onto themselves to fasten the strap 18A between the mounting devices 22 and 32, respectively. Given equal tension load carrying capability, the strap design of the tension bearing element may be configured to have a lower bending stiffness than the cylindrical cable of Figure 1. However, the strap design may require more care during installation to assure that the bending planes of composite flexure pairs 10 are aligned with each other. The other parts of this arrangement are the same as and function the same way as described in connection with Figure 1.

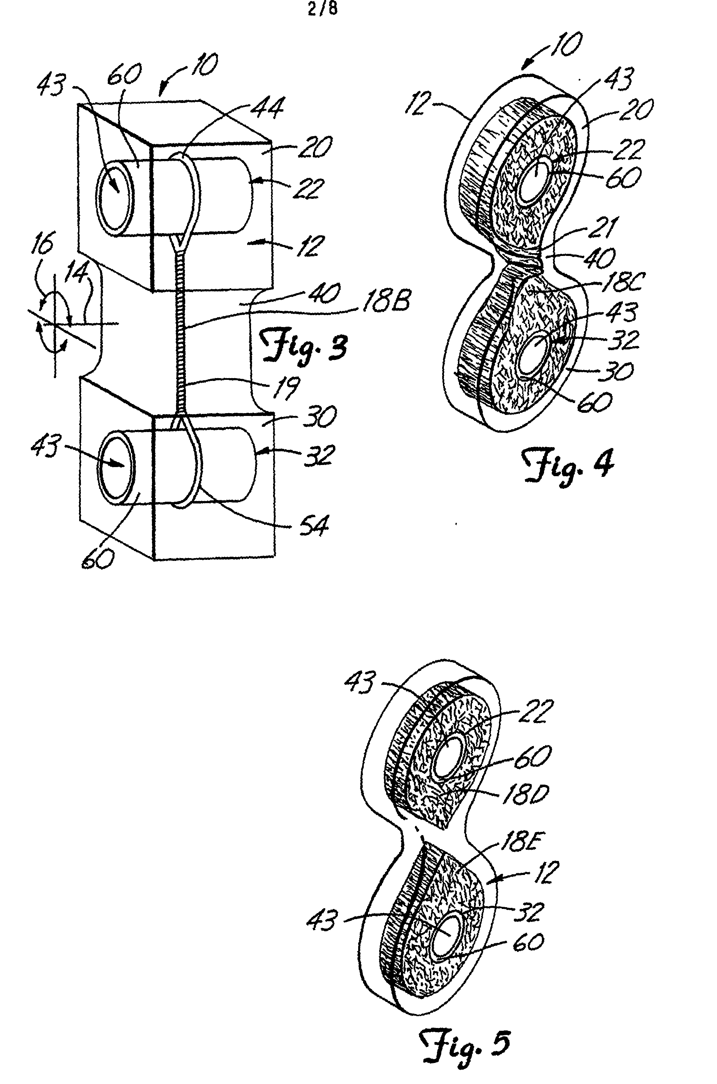

In Figure 3, the load bearing element is shown at 18B and is formed by a tension winding of carbon, glass or other filaments around the shock absorbing sleeves 60, and then a winding of a finished circumferential bundling or over wrap 19 achieves the desired tension carrying capabilities between the first and second mounting devices 22 and 32. The other parts of this arrangement are the same as and function the same way as described in connection with Figure 1.

In Figure 4, the tension load bearing element 18 is a fiber insert 18C which is placed in a mold and impregnated with polyurethane during injection molding of the preferred polyurethane for the flexure unit 10. The fiber insert 18C is twisted at 21 so that the requisite tension carrying ability is achieved between the first and second mounting devices 22 and 32. The twisting of the fiber insert 18C helps pack and confine the central area fibers close to the center of the third portion 40 of the flexure 12. This is a very inexpensive way to preferentially add tensile strength to a short flexure unit. The other parts of this arrangement are the same as and function the same way as described in connection with Figure 1.

Figure 5 shows a variation on the arrangement of Figure 4 in which fiber inserts 18D and 18E are formed as end discs positioned only around the sleeves 60 of the first and second mounting devices 22 and 32. Each disc extends only partially into the, third portion 40 of the flexure 12. The discs are not connected to each other. The fiber inserts 18D and 18E increase the strength and rigidity selectively in the areas of fastening so that the flexure 12 material thickness and bulk around the sleeves 60 forming mounting holes 43 can be reduced without increasing flexure/bending rigidity in the third portion 40 of the flexure 12 (See also Figures 10 and 11) and to reduce progressive elongation and tearing around the areas of fastening. In this arrangement the load bearing elements are discs 18D and 18E which selectively increase the strength and rigidity of the flexure 12 at the first and second fastening devices 22 and 32, however, other variations will be apparent to those skilled in the art.

Referring to Figures 6-9, the orthotic shell 28 or other structure to which the composite flexure units 10 are mounted may be configured to surround or wrap around an outer surface of the composite flexure 10 so as to reduce the magnitude of unwanted modes of transverse shear deflections and rotation. In orthotic applications, the orthotic shell 28 has a recess 72 (See Figures 8 and 9) for receiving the ends of composite flexure unit 10. The first and second portions 20 and 30 of the flexure 12 are surrounded and restrained by the surfaces forming recess 72 on the front 73, back 74, and side 75 surfaces of the flexure 12, as shown in Figure 8. A cover plate 80 comprised of a high modulus of elasticity material completes the surround/restraint function of the orthosis shell 28. The cover plate 80 covers an outer surface 76 of each of the ends of flexure 12 to further reduce the unwanted translational and rotational motions of the flexure 12 without restricting motion in the bending plane 16. The cover plates 80 are installed as part of the mounting devices 22 and 32 to spread the loads created by the flexure 12 on the mounting device 22 and 32 over a greater area. The cover plates 80 may be etched away or relieved as shown at 80A on either the front or rear in the third portion 40 of the flexure 12 to restrict planterflexion or dorsiflexion (See Figures 7 and 9) by providing stop surfaces. The cover plates may be etched away or relieved as shown at 80B on both the front and rear in the third portion 40 (See Figure 6) to permit two way flexion or reverse bending.

Figures 10 and 11 show a preferred embodiment in which the flexure 12 is arched and the thickness of the first and second portions 20 and 30 of the flexure 12 are reduced beyond the first and second mounting devices 22 and 32 and are tapered as shown.

Referring to Figure 10, cover plates 80C are shown with impinging anchor points 82 for engaging an outer surface 84 of the orthosis shell 28, in cases where the shell 28 does not have the recess 72. In Figure 11, a cover plate 80D is shown with flared, serrated edges 86 which impinge the flexure 12 and anchor the cover plate 80D to an inner surface 88 the orthotic shell 28 in the recess 72B. Figures 10 and 11 both show ways to distribute the tension loads across a greater area of the orthosis shell 28.

Another embodiment of the composite flexure unit 10' of the present invention is illustrated in Figure 12. The load bearing element 18F is not encapsulated along the centerline of the third portion 40 of the flexure 12', rather it is offset significantly to one side of the portion 40A. The load bearing element 18F creates a bending compliance bias between dorsiflexion and planterflexion. The degree of bias may be varied by a tension adjustment mechanism 90 before, during, and/or after installation of the composite flexure unit 10' to create a bending bias on the flexure 12'. The tension adjustment mechanism 90 includes a loop portion 92 which is looped around and fixed to the sleeve 60 of the first mounting device 22 and a tightening member 94 for shortening the tension member 18F and thereby biasing the load bearing member 18F. The tightening member 94 is accessible from the outer surface of the flexure 12' and may be a cam or a gear driven screw combination for adjusting the tension. Another practical approach to varying the bias would be to produce flexure units with various amounts of non-adjustable bias.

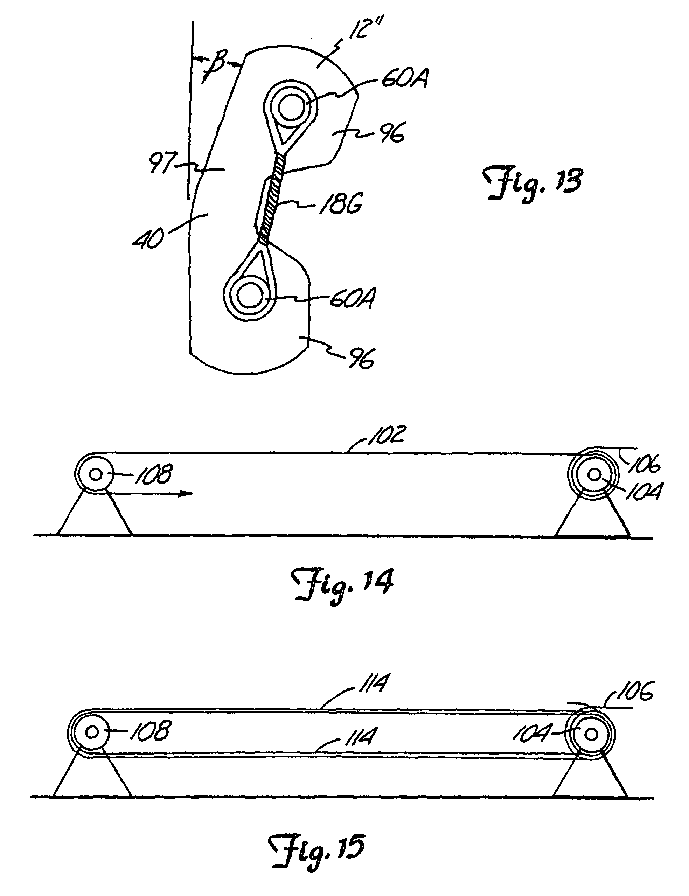

In one such practical approach for providing a flexure unit with non-adjustable pretension is illustrated in Figure 13. Flexure 12" has a load bearing element 18G, which would be constructed similarly to load bearing element 18F, which is not encapsulated along the center portion of the third portion 97 of the flexure of 12". The third portion 97 is offset significantly to one side of the end portions 96 of the flexure. The "at rest" configuration of the flexure with the load bearing element 18G at rest is bowed. The angle associated with that bow is indicated as β in Figure 13. If the angle β corresponds to a non-neutral ankle flexion angle of an ankle-foot orthosis, one angular motion from the neutral ankle position, dorsiflexion for instance, will be assisted and the opposite angular motion, plantarflexion, will have a preloaded resistance. The molded configuration of center portion 97 will be forced to straighten and compress when the hinge to which it is attached is in a position in which the flexure 12" is forced into a β = 0° alignment. This will create a preload tension in the load bearing element 18G and a preload compression in the polyurethane section 40 that would, for example, be useful in providing "toe lift".

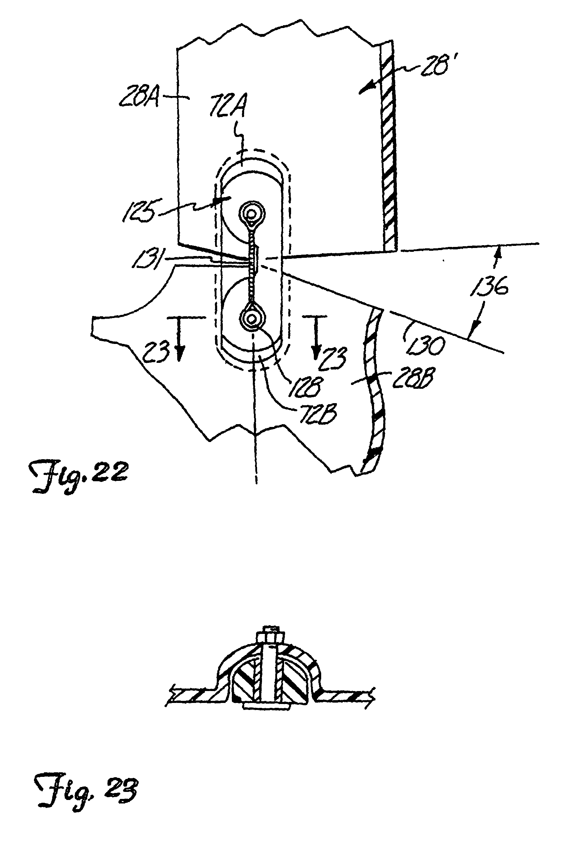

It is possible to create an equivalent type of joint motion bias by using a "molding dummy" with a configuration equivalent to 12" when creating the mounting cavity in the plastic shell parts 28. If the molding dummy is configured with β angle of -25°, for instance, as shown in Figures 22 and 23 the flexure, with offset tension element, has a β angle of 0°, for instance, a bias is achieved. By choosing one of several β angle differences between molding dummy and operational flexure, one can choose different levels of preload and angular motion bias.

When used in pairs to create an orthotic or other joint (or hinge), rotation of the composite flexure unit 10', shown in Figure 12, about the rotational axis 14 may be assisted or resisted. Rotational motion stops 95 (see Figures 6 and 7) formed by abutting surfaces on the two relatively movable parts of the orthotic shell 28 may be used if desired with the biased (or biasable) composite flexure unit pairs 10' to create virtually any desired combination of rotational motion management. The stops also can be eliminated if desired. Tensioning or shortening the tensile load bearing element 18F with the tension adjustment mechanism 90 when the element 18F is positioned posterior of the portion 14A of flexure 12' would create a planterflexion assist bias. If the composite flexure 10' was reversed 180° so that the load bearing element 18F was positioned anterior of the portion 14A of low modulus flexure 12', tensioning or shortening will create a dorsiflexion assist bias. Thus, for example, by managing installation alignment of the composite flexure, such that the composite flexure unit 10' is biased for dorsiflexion and by placing a stop on the parts of the orthosis shell 28 to prevent planterflexion, the composite flexure unit 10' may function like a spring-loaded hinge, biased to assist dorsiflexion and block plantarflexion. Other combinations of ankle motion assistance and limitation are easily achieved.

The same effects can be obtained with the embodiment of Figure 13, except the tension load on load bearing element 18G would be provided at an initial setting and would not be adjustable.

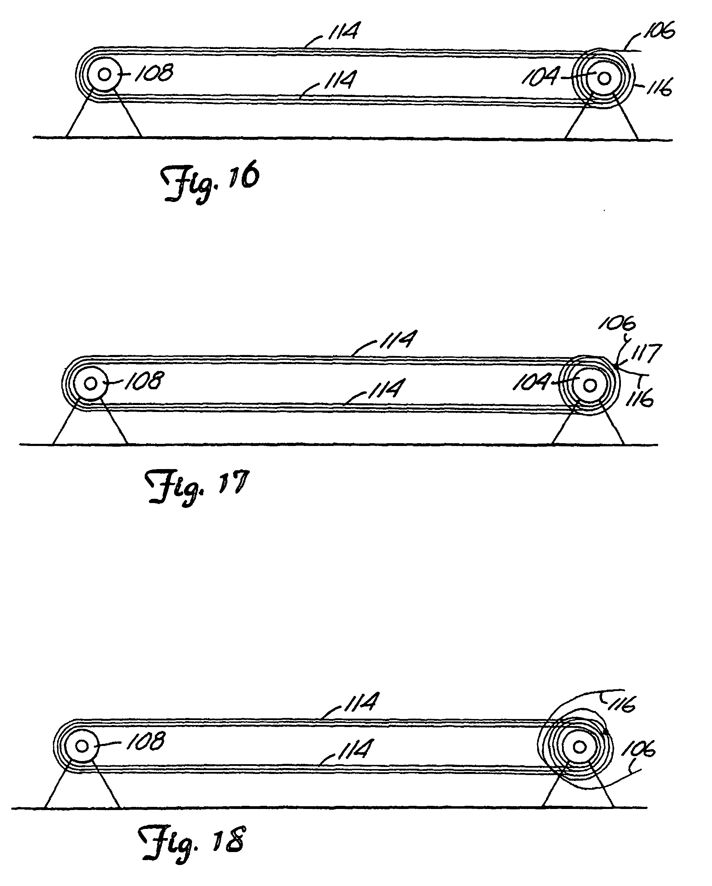

In Figures 14 through 21 a further composite flexure unit 100 is illustrated. The composite flexure unit 100 is preferably made with a load bearing element of strands of fibers or threads of oriented tetrafluoroethylene, sold under the trademark TEFLON. Oriented Teflon is essentially a fine Teflon fiber or thread that is stretched under conditions that orient the molecules along the length of the fiber to reduce its elongation characteristics under load and increase the tensile elastic modulus and strength of the fiber. The material is commercially available and the diameter size can vary. However, the thread preferably is quite fine for the application shown, to reduce the overall size of the composite flexure 100 while maintaining adequate tensile strength. One existing use for this fiber or thread is as a dental floss sold by Colgate-Palmolive Co.

A length of fiber or thread 102 is wrapped over a first sleeve 104 that is to be used for mounting one end of the finished flexure 100 in position on an orthotic of other joint or hinge. A free end 106 is left extending out for tie off and three dr so wraps are made of the fiber as a first step to insure that the free end will not slip off the first sleeve. The fiber is then passed over a second sleeve 108 which is to be used for mounting the second end of the finished flexure 100. The sleeves are mounted on suitable supports 110 and the proper distance apart and one end of each sleeve is left open or uncovered for wrapping.

As shown in Figure 15 the fiber is then wrapped around the two sleeves a number of times to build up the size of the multi-strand lengths 114 of the fiber extending between the two sleeves to the desired size. The free end of the fiber is then wrapped a few times around the sleeve 104, trimmed if necessary, and then the second end 116 of the fiber is tied off with the end 106, in a knot 117.

The ends of the thread are left long enough so after the first tie off shown in Figure 17, the ends are reverse wrapped (wrapped in the opposite direction) as shown in Figure 18 and tied again. The exact tie off can be varied as needed, but it is important that the fiber or thread does not slip and unwrap.

The fiber lengths are kept under a tension load during the wrapping process to insure that the fiber strands forming lengths 114 nest and stay straight to avoid any slack and insure that the strands of each of the lengths 114 carry a tension load when in use. The preferred material, Teflon, is very slippery and thus the tying has to be carefully done. The fiber ends could be secured in other ways, such as pinning to the sleeves or using small clamp bands around the sleeve 104.

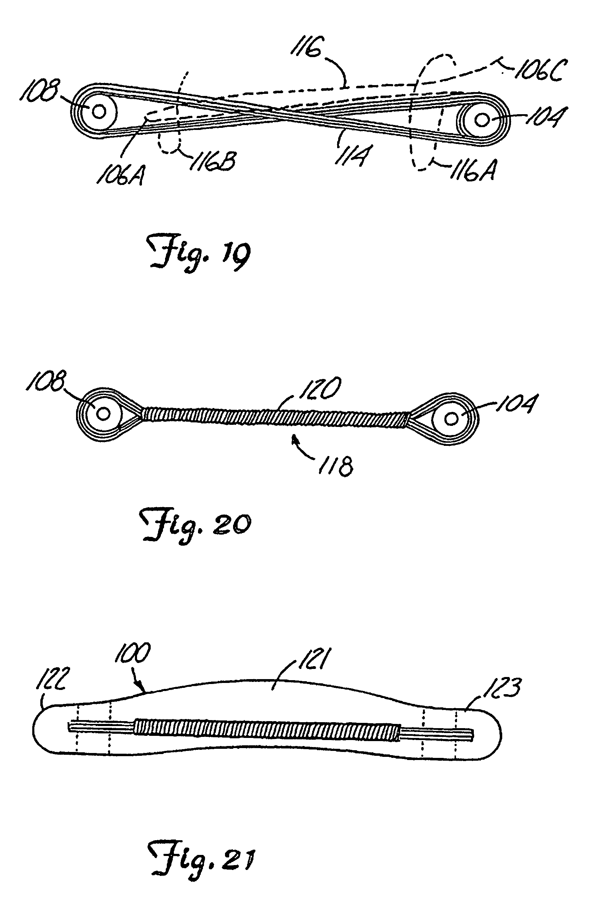

After the fiber is wrapped a desired number of times, for example to form 20 to 30 strands of thread in each length 114, one of the two sleeves, 108 or 104 is removed from the mounting peg on its support 110, rotated 180° and placed back on the peg to produce the "figure-of-eight" configuration shown in Figure 19. Then the free end 116 of the fiber is wound circumferentially around the fibers of the two lengths 114 to create a "bundled" configuration 120 as illustrated in Figure 20 and the end 116 suitably secured.

The finished tension load bearing element (Figure 20), including the two sleeves (104 and 108) is placed in a suitable fixture and encapsulated in a low modulus of elasticity material, such a polyurethane, shown at 121. The preferred configuration of the finished composite flexure unit 100 is as shown in Figure 21.

Another way of tieing off the ends of the fibers after the continuous wrap of the lengths 114, is illustrated in dotted lines in 18. The free end 106 is left long enough so it can be laid as shown in dotted lines along one of the lengths 14. The length 106 is looped as shown at 106A so that the free end 106B is doubled back along length 114. As the wrapping end 116 of fiber is wrapped around the Figure 8 lengths 114, 114, the wrapping commences adjacent the sleeve 104 and the free end 116 is wrapped onto the two lengths 114 and the looped lengths of fiber end 106. When the wrap is almost to the loop end 106A, as shown on the line generally at 116B, the free end 116 that is used for making the overwrap is passed through this loop at 106A, and then the opposite free end 106B will be pulled to pull the loop end 106A and a portion of the free end fiber 116 underneath the overwrap 120. This will anchor both of the free ends 106 and 116 under the overwrap 120, and insure a secure non-slip binding.

The sleeves or mounting devices 104 and 108 are used for attachment in position on an orthotic joint such as that shown in Figures 6 and 7, or in some other joint or hinge, using suitable pins passing through the center openings in the sleeves. The load bearing element 118 made of oriented Teflon has excellent tension carrying abilities with low mass and size, and does not stretch significantly under load. Oriented Teflon has a high modulus of elasticity to provide longitudinal strength and stiffness. When bent about the flexion axis, the stranded load bearing element 118 does not increase the flexion stiffness significantly. The crossing of the lengths 114 after being formed into the figure 8 places the bending axis adjacent to or on the crossing point where the bending stiffness of the load bearing element 118 is the lowest.

Teflon has a low coefficient of friction, as is well known, and since the individual threads or fibers are not attached to each other they will slip past each other when the load bearing element 118 is flexed. Therefore there is little abrasion, which results in long life of the load bearing element. The multiple fibers oriented Teflon forming the load bearing element also are strong and since the fibers will slip past each other, each strand of fiber or thread will share load. During bending flexion, the low friction coefficient of Teflon, and the inter-fiber sliding that is permitted, will allow each fiber to bend independently. The bundle will not bend as a beam subjecting surface strands to higher stresses than the more centered strands. Further, in the Figure 8 configuration the load on each fiber or thread will equalize on opposite sides of the bend.

Teflon also can be encapsulated in thermoplastic materials, and specifically the Teflon load bearing element can be molded inside polyurethane. Here again the characteristics of Teflon are helpful. The lack of a firm bond between the Teflon and encapsulating polyurethane allows the tension element to bend as a bundle of fibers rather than as a bonded-in part of a composite beam. Polyurethane has a high molding temperature which would tend to anneal many plastic fibers. However, Teflon will withstand injection molding temperatures needed for Polyurethane without losing the molecular orientation and corresponding high strength gained during the stretching part of the fiber manufacturing process.

Other fibers or threads of similar characteristics can be used, such as nylon and Dacron polyester fibers. However, these fibers tend to stretch and do not have the ability to withstand injection molding temperatures.

Figures 22 and 23 show an orthotic shell 28' or other structure, to which the composite flexure units 10 are mounted, configured to provide a recess for receiving a flexure unit. This is similar to the construction shown in Figures 6-9. In Figure 22, the upper portion 28A of the orthotic shell 28' has a molded-in recess 72A and the lower portion 28B of the orthotic shell 28' has a recess 72B. These recesses are of size to receive the ends of a composite flexure unit 125, constructed such as that shown at 10' in Figure 12, or the unit shown in Figure 13 with β equal to 0.

End portions 126A and 126B of the composite flexure unit 125 are surrounded on three sides by the surfaces forming the recess, and are secured in place with a suitable pin 128.

The recesses 72A and 72B are created with the use of a molding dummy configured with a β angle equal to a minus value, -25° for instance. This angle, -25° for instance, corresponds to the angle indicated by the line with double arrows at 131. A composite flexure unit that has load bearing member offset from the center portions of the flexure unit as shown is formed to be "at rest" with the flexure straight as shown, the offset angle of the recess 72B will cause the forward end of the lower shell portion 28B to tilt upwardly and provide a bias force useful for "toe lift". In use when the lower shell is supporting a foot, the shell rear parts will move toward or to the dotted line position shown in Figure 22 and this will provide a preload on the load bearing member shown at 131 in Figure 22. As stated previously, choosing one of several different angle differences between the angle of the center line of one or both recesses formed by a molding dummy when the orthotic shell is molded, and the angle β of the flexure, which is shown in Figure 13, one can choose different levels of preload and angular motion bias.

Although the present invention has been described with particular application in the orthotics industry, there are applications in many products, structures, and mechanisms where the range of rotational motion consists of an arch of less than 360 degrees and where a conventional hinging components are undesirable because of wear characteristics, weight, complexity.

## Claims

1. A composite flexure unit comprising
a flexure member (100) including a first portion (122) having first mounting means (108) therein for mounting a first end of the flexure member (100), a second portion (123) having second mounting means (104) therein for mounting a second end of the flexure member (100), and a third portion (121) connecting the first portion (122) to the second portion (123), the flexure member (100) being operable for bending about a rotational axis passing through the third portion (121) of the flexure member (100); and
a load bearing element (118) connected between the first (108) and second (104) mounting means;
the flexure member (100) having a low modulus of elasticity and the load bearing element (118) having a high modulus of elasticity for providing longitudinal strength and stiffness to the flexure member (100) without significantly increasing flexion stiffness about the rotational axis, the load bearing element bending with the flexure member as the flexure member bends;
**characterised in that** the load bearing element (118) comprises a multi-strand element (118) formed of a fiber having a low coefficient of friction, the fiber being wrapped around and extending between end sleeves (104, 108) forming the mounting means (104, 108) to form two multi-strand lengths (114) in which the fiber strands (102) are free to slide relative to other fiber strands in the same multi-strand length (114).

2. The composite flexure unit of claim 1, wherein the load bearing element (118) is mounted within the first (122), second (123) and third (121) portions of the flexure member (100).

3. The composite flexure unit of any preceding claim, wherein the two multi-strand lengths (114) extend between the end sleeves (104, 108), and cross in center portions between the end sleeves (104, 108) to form a figure 8 pattern.

4. The composite flexure unit of any preceding claim, further comprising an overwrap of a fiber length (116) around the multi-strand lengths (114) along a major portion (120) of the distance between the end sleeves (104, 108).

5. The composite flexure unit of any preceding claim, wherein the load bearing element (118) comprises oriented tetrafluoro-ethylene.

6. The composite flexure unit of any preceding claim, further comprising a first cover plate (80) of a high modulus of elasticity material positioned on the first portion (122) of the flexure member (100), and a second cover plate (80) of a high modulus of elasticity material positioned on the second portion (123) of the flexure member (100), the first and second cover plates (80) restricting bending and shear to occur only in the third portion (121) of the flexure member (100), and being operable with the first (108) and second (104) mounting means, respectively, for spreading loads created by the load bearing element (118) to a greater area defined by the first and second cover plates (80).

7. The composite flexure unit of any preceding claim in combination with an orthotic shell (28) having two parts that are held for pivoting movement by the flexure member (100) and means (41, 42) for mounting the first and second portions of the flexure member (100) to the two parts, the mounted flexure member being oriented to provide a bias in one direction of pivoting from a reference position.

8. The combination of claim 7, in which the mounting of the flexure member comprises a recess (72) in the shell (28) that has an axis that passes through the first (122) and second (123) portions of the flexure member (100) when the shell portions (80) are mounted, said axis being angled relative to a reference line of the shell portions (80) to provide a bias when the flexure member (100) is mounted on the shell portions (80) relative to the reference line.

## Patentansprüche

1. Zusammengesetzte Biegungseinheit, welche Folgendes aufweist:
ein Biegungselement (100), das einen ersten Abschnitt (122), der eine erste Befestigungseinrichtung (108) zum Befestigen eines ersten Endes des Biegungselements (100) besitzt, einen zweiten Abschnitt (123), der eine zweite Befestigungseinrichtung (104) zum Befestigen eines zweiten Endes des Biegungselements (100) aufweist, und einen dritten Abschnitt (121) aufweist, der den ersten Abschnitt (122) mit dem zweiten Abschnitt (123) verbindet, wobei das Biegungselement (100) um die durch den dritten Abschnitt (121) des Biegungselements (100) hindurchgehende Drehachse biegbar ist; und
ein Lasten-Tragelement (118), das zwischen der ersten (108) und der zweiten (104) Befestigungseinrichtung angeschlossen ist;
wobei das Biegungselement (100) einen niedrigen Elastizitätsmodul und das Lasten-Tragelement (118) einen hohen Elastizitätsmodul aufweist, um dem Biegungselement (100) Längsfestigkeit und Steifigkeit zu verleihen, ohne dass die Biegesteifigkeit um die Drehachse erheblich erhöht wird, wobei sich das Lasten-Tragelement mit dem Biegungselement biegt, wenn sich dieses biegt;
**dadurch gekennzeichnet, dass** das Lasten-Tragelement (118) ein Multi-Litzenelement (118) aufweist, das aus einer Faser mit einem niedrigen Reibungskoeffizienten gebildet ist, wobei die Faser um Endmuffen (104, 108) gewickelt ist bzw. sich zwischen diesen erstreckt und die Endmuffen die Befestigungseinrichtungen (104, 108) schaffen, um zwei Multi-Litzenlängenstücke (114) zu bilden, in denen die Faserlitzen (102) frei relativ zu anderen Faserlitzen mit der gleichen Multi-Litzenlänge (114) gleiten können.

2. Zusammengesetzte Biegungseinheit nach Anspruch 1, **dadurch gekennzeichnet, dass** das Lasten-Traglement (118) innerhalb des ersten (122), des zweiten (123) und des dritten (121) Abschnitts des Biegungselements (100) angebracht ist.

3. Zusammengesetzte Biegungseinheit nach einem der vorhergehenden Ansprüche, **dadurch gekennzeichnet, dass** sich die beiden Multi-Litzenlängenstücke (114) zwischen den Endmuffen (104, 108) und quer in den Mittelabschnitten zwischen den Endmuffen (104, 108) erstrecken, um ein Muster in Form der Zahl 8 zu bilden.

4. Zusammengesetzte Biegungseinheit nach einem der vorhergehenden Ansprüche, welche ferner ein Hüllelement eines Faserlängenstücks (116) um die Multi-Litzenlängenstücke (114) herum entlang einem Hauptabschnitt (120) des Abstands zwischen den beiden Endmuffen (104, 108) aufweist.

5. Zusammengesetzte Biegungseinheit nach einem der vorhergehenden Ansprüche, **dadurch gekennzeichnet, dass** das Lasten-Tragelement (118) gerecktes Tetrafluor-Ethylen aufweist.

6. Zusammengesetzte Biegungseinheit nach einem der vorhergehenden Ansprüche, welche ferner eine erste Abdeckplatte (80) aus einem Werkstoff mit einem hohen Elastizitätsmodul aufweist, die auf dem ersten Abschnitt (122) des Biegungselements (100) angeordnet ist, sowie eine zweite Abdeckplatte (80) aus einem Werkstoff mit einem hohen Elastizitätsmodul, die auf dem zweiten Abschnitt (123) des Biegungselements (100) angeordnet ist, wobei die erste und die zweite Abdeckplatte (80) Biegung und Scherung so begrenzen, dass sie nur in dem dritten Abschnitt (121) des Biegungselements (100) auftreten, und zusammen mit der ersten (108) bzw. der zweiten (104) Befestigungseinrichtung jeweils betriebsbereit sind, um von dem Lasten-Tragelement (118) erzeugte Belastungen auf eine größere Fläche zu verteilen, wobei die Fläche durch die erste und zweite Abdeckplatte (80) definiert ist.

7. Zusammengesetzte Biegungseinheit nach einem der vorhergehenden Ansprüche in Kombination mit einer orthetischen (stützend haltungskorrigierend) Schale (28), welche zwei Teile aufweist, die für die Schwenkbewegung durch das Biegungselement (100) gehalten werden, und Einrichtungen (41, 42) zum Befestigen des ersten und des zweiten Abschnitts des Biegungselements (100) an den beiden Teilen, wobei das angebrachte Biegungselement so ausgerichtet ist, dass es eine Vorspannung in eine Schwenkrichtung von einer Bezugsposition aus liefert.

8. Die Kombination von Anspruch 7, bei welcher der Aufbau des Biegungselements eine Aussparung (72) in der Schale (28) aufweist, die eine Achse besitzt, welche durch den ersten (122) und den zweiten (123) Abschnitt des Biegungselements (100) hindurchgeht, wenn die Schalenabschnitte (80) befestigt sind, wobei die Achse in einem Winkel relativ zu einer Bezugslinie der Schalenabschnitte (80) angeordnet ist, um eine Vorspannung zu erzeugen, wenn das Biegungselement (100) auf den Schalenabschnitten (80) relativ zu der Bezugslinie angebracht wird.

## Revendications

1. Une unité de flexion composite comprenant
un organe de flexion (100) comprenant une première partie (122) ayant en son sein des premiers moyens de montage (108) pour monter une première extrémité de l'organe de flexion (100), une deuxième partie (123) ayant en son sein des deuxièmes moyens de montage (104) pour monter une deuxième extrémité de l'organe de flexion (100), et une troisième partie (121) reliant la première partie (122) à la deuxième partie (123), l'organe de flexion (100) étant susceptible de fonctionner pour fléchir autour d'un axe de rotation passant par la troisième partie (121) de l'organe de flexion (100) ; et
un élément support de charge (118) relié entre les premier (108) et deuxième (104) moyens de montage ;
l'organe de flexion (100) ayant un bas module d'élasticité et l'élément support de charge (118) ayant un haut module d'élasticité pour fournir une résistance et une rigidité longitudinale à l'organe de flexion (100) sans significativement augmenter la rigidité en flexion autour de l'axe de rotation, l'élément support de charge fléchissant avec l'organe de flexion lors de la flexion de cet organe de flexion ;
**caractérisée en ce que** l'élément support de charge (118) comprend un élément multibrins (118) formé de fibres ayant un faible coefficient de friction multibrins, enroulées autour et s'étendant entre des manchons d'extrémité (104, 108) formant les moyens de montage (104, 108) pour former deux longueurs multibrins (114), dans lesquelles les brins à fibre (102) sont libres de coulisser les uns par rapport aux autres brins de fibre, dans la même longueur de multibrins (114).

2. L'unité de flexion composite selon la revendication 1, dans laquelle l'élément support de charge (118) est monté à l'intérieur des première (122), deuxième (123) et troisième (121) parties de l'organe de flexion (100).

3. L'unité de flexion composite selon l'une quelconque des revendications précédentes dans laquelle les deux longueurs multibrins (114) s'étendent entre les manchons d'extrémité (104, 108) et se croisent en des parties centrales entre les manchons d'extrémité (104, 108) pour faire un motif en forme de chiffre 8.

4. L'unité de flexion composite selon l'une quelconque des revendications précédentes comprenant en outre un surenveloppement d'une longueur de fibre 116 autour des longueurs multibrins (114), sur une partie principale (120) de la distance entre les manchons d'extrémités (104, 108).

5. L'unité de flexion composite selon l'une quelconque des revendications précédentes, dans laquelle l'élément support de charge (118) est composé de tétrafluoro-éthylène orienté.

6. L'unité de flexion composite selon l'une quelconque des revendications précédentes comprenant en outre une première plaque de couverture (80) en un matériau à haut module d'élasticité, placée sur la première partie (122) de l'organe de flexion (100), et une deuxième plaque de couverture (80) en un matériau à haut module d'élasticité, placée sur la deuxième partie (123) de l'organe de flexion (100), les première et deuxième plaques de couverture (80) restreignant la flexion et le cisaillement pour qu'ils ne produisent que dans la troisième partie (121) de l'organe de flexion, et étant susceptibles de fonctionner avec les premiers (108) et deuxième (104) moyens de montage, respectivement, pour disperser les charges créées par l'organe support de charge (118) sur une plus grande surface, définie par les première et deuxième plaques de couverture (80).

7. L'unité de flexion composite selon l'une quelconque des revendications précédentes en combinaison avec une enveloppe orthotique (28) comportant deux parties, maintenues pour permettre un mouvement pivotant par l'organe de flexion (100), et des moyens (41, 42) pour monter les premières et deuxièmes parties de l'organe de flexion (100) sur les deux parties, l'organe de flexion, ayant été monté, étant orienté pour fournir un déplacement dans un sens de pivotement, depuis une position de référence.

8. La combinaison selon la revendication 7 dans laquelle le montage de l'organe de flexion comprend une cavité (72), dans l'enveloppe (28), qui a un axe passant à travers les première (122) et deuxième (123) parties de l'organe de flexion (100) lorsque les parties d'enveloppe (80) sont montées, ledit axe étant incliné par rapport à une ligne de référence des parties d'enveloppe (80), pour foumir une déformation lorsque l'organe de flexion (100) est monté sur les parties d'enveloppe (80) par rapport à la ligne de référence.
